# EUROPÄISCHE PATENTSCHRIFT

(11) **EP 0 616 642 B1**
(45) Veröffentlichungstag und Bekanntmachung des Hinweises auf die Patenterteilung: **05.02.1997**
(21) Anmeldenummer: 92924598.3
(22) Anmeldetag: 19.11.1992
(51) Int. Cl.: C12N 15/15, C07K 14/00, A61K 38/55

(54) **NEUE THROMBININHIBITORISCHE PROTEINE AUS LANDBLUTEGELN**
NEW THROMBIN-INHIBITING PROTEINS FROM TERRESTRIAL LEECHES
NOUVELLES PROTEINES INHIBITRICES DE THROMBINE PROVENANT DE LA SANGSUE DES CAMPAGNES

(30) Priorität: 26.11.1991 DE 4138698; 20.03.1992 DE 4209110
(43) Veröffentlichungstag der Anmeldung: 28.09.1994
(73) Patentinhaber: BASF Aktiengesellschaft, 67063 Ludwigshafen (DE)
(72) Erfinder: STRUBE, Karl-Hermann, D-6720 Speyer (DE); BIALOJAN, Siegfried, D-6836 Oftersheim (DE); KROEGER, Burkhard, D-67117 Limburgerhof (DE); FRIEDRICH, Thomas, D-6100 Darmstadt (DE)
(86) Internationale Anmeldenummer: EP9202661
(87) Internationale Veröffentlichungsnummer: WO9311239

(56) Entgegenhaltungen:
- FEBS LETTERS. Bd. 255, Nr. 1, 11. September 1989, AMSTERDAM NL Seiten 105 - 110 M. SCHARF ET AL 'Primary structures of new iso-hirudins'

## Beschreibung

Die vorliegende Erfindung betrifft neue thrombininhibitorische Proteine aus dem Landblutegel Haemadipsa sylvestris sowie Verfahren zu deren Herstellung.

Thrombin-Inhibitoren sind wichtige therapeutische Substanzen, die beispielsweise zur Prophylaxe oder Behandlung von Thrombosen oder arteriellen Reokklusionen verwendet werden.

In der EP 142 860 wird der Thrombininhibitor Hirudin aus dem medizinischen Blutegel (Hirudo medicinalis) mit seiner Primarstruktur beschrieben. Weiterhin ist die gentechnische Herstellung von Hirudin beispielsweise aus EP 168 342 bekannt.

Es wurden nun neue thrombininhibitorische Proteine aus dem Landblutegel Haemadipsa sylvestris isoliert.

Die neuen Proteine besitzen folgende physikochemischen Eigenschaften. Durch Molekularsiebchromatographie wird ihnen ein Molekulargewicht von 11200 ± 1000 Dalton zugeordnet. In einem SDS-Polyacrylamidgel wird ein Molekulargewicht von 5000 ± 1000 Dalton bestimmt.

Die Proteine binden spezifisch an eine Thrombin-Affinitatssaule. Sie hemmen die biologische Aktivität von Thrombin in einem in-vitro Enzymtest.

Folgende N-terminale Aminosäuresequenz wurde von den Proteinen bestimmt (SEQ ID NO: 3): worin A für Asp oder Phe, B für Glu oder Trp, C für Lys, Asn oder Asp und D für Pro oder Leu steht.

Die neuen Proteine lassen sich aus Landblutegeln der Gattung Haemadipsa isolieren. Hierzu werden die Egel zweckmäßigerweise in einem Puffer bei pH 6 bis 9, vorzugsweise pH bis 8 aufgenommen und mit einem Homogenisator, vorzugsweise einem Mixer, homogenisiert. Anschließend werden die unlöslichen Bestandteile abgetrennt, bevorzugt abzentrifugiert.

Aus der so erhaltenen Lösung können die Proteine weiter durch chromatographische Methoden, bevorzugt Ionenaustauschchromatographie und/oder Affinitätschromatographie, gereinigt werden. Besonders bevorzugt ist ein Reinigungsschritt über Thrombin-Affinitätschromatographie.

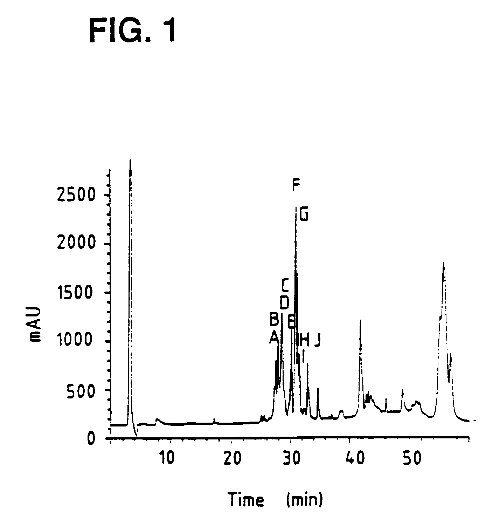

Nach Affinitätschromatographie an einer Thrombin-Säule können durch reversed phase HPLC verschiedene Isoproteine mit thrombininhibitorischer Aktivität getrennt erhalten werden (s. Fig. 1).

Die Reinigung der Proteine kann über einen Thrombin-Aktivitätstest verfolgt werden. Hierzu benutzt man zweckmäßigerweise einen optischen Test, bei dem ein chromogenes Substrat, beispielsweise Chromozym TH, durch Thrombin umgesetzt wird. Die die neuen Proteine enthaltenden Fraktionen können bei Zugabe in diesen optischen Test an ihrer Thrombin inhibierenden Wirkung erkannt werden.

Besonders geeignet zur Herstellung der erfindungsgemäßen Proteine sind gentechnische Verfahren.

Hierzu wird in an sich bekannter Weise eine cDNA-Genbank aus dem Egel angelegt. Aus dieser Genbank kann das für das erfindungsgemäße Protein codierende Gen isoliert werden, indem man beispielsweise eine DNA-Probe herstellt, deren Sequenz von der oben beschriebenen N-terminalen Aminosäuresequenz durch Rückübersetzung gemäß dem genetischen Code erhalten wird. Durch Hybridisierung mit dieser DNA-Probe läßt sich das entsprechende Gen auffinden und isolieren.

Für die Herstellung des entsprechenden Gens kann aber auch die Polymerase-Chain-Reaction (PCR)-Technik eingesetzt werden. Beispielsweise läßt sich mit Hilfe eines Primers, dessen Sequenz durch Rückübersetzung aus der oben beschriebenen N-terminalen Aminosäuresequenz erhalten wurde, und eines zweiten Primers, dessen Sequenz komplementär zum 3'-Ende des cDNA-Genfragments ist, bevorzugt mit der Sequenz Poly(dT), das cDNA-Genfragment für das erfindungsgemäße Protein durch PCR-Technik herstellen. Das entsprechende Gen läßt sich auch isolieren, indem man eine Expressionsgenbank von Egeln anlegt und diese mit einem Antikörper, der gegen das erfindungsgemäße Protein gerichtet ist, absucht.

Eine cDNA, die für ein erfindungsgemäßes Protein codiert, ist in SEQ ID NO 22 dargestellt.

Weitere geeignete DNA-Sequenzen sind solche, die zwar eine andere Nukleotidsequenz als die in SEQ ID NO 22 aufgeführte besitzen, die aber infolge der Degeneration des genetischen Codes für die in SEQ ID NO 22 aufgeführte Polypeptidkette oder Teile davon codieren. Weiterhin sind solche DNA-Sequenzen geeignet, die für Proteine mit thrombininhibitorischer Wirkung codieren, und die unter Standardbedingungen mit der in SEQ ID NO 22 dargestellten Nukleotidsequenz oder mit einer Nukleotidsequenz, die für das in SEQ ID NO 22 dargestellte Protein codiert, hybridisieren. Die experimentellen Bedingungen für DNA-Hybridisierung sind in Lehrbüchern der Gentechnik, beispielsweise in Maniatis et al., "Molecular Cloning", Cold Spring Harbor Laboratory, 1990, beschrieben.

Unter Standardbedingungen sind beispielsweise Temperaturen zwischen 42 und 58°C in einer wäßrigen Pufferlösung mit einer Konzentration zwischen 0,1 und 1 x SSC (1 x SSC: 0,15M NaCl, 15mM Natriumcitrat pH 7,2) zu verstehen.

Nachdem das entsprechende Gen isoliert worden ist, kann es durch gentechnische Verfahren in Organismen, z.B. in Bakterien, Hefen oder höheren eukaryontischen Zellen, mit Hilfe eines Expressionsvektors in an sich bekannter Weise exprimiert werden.

Bevorzugt werden prokaryontische Expressionssysteme verwendet, die die erfindungsgemäßen Proteine in Form von Fusionsproteinen synthetisieren. Ein Beispiel für ein solches Expressionssystem ist das kommerziell erhältliche Profusion®-System (New England Biolabs), das das gewünschte Protein als Fusionsprotein mit dem Maltose-Bindungsprotein unter der Kontrolle des induzierbaren tac-Promotors synthetisiert.

Aus dem Fusionsprotein kann das gewunschte Protein durch Spaltung mit Faktor Xa freigesetzt werden.

Aus diesen rekombinanten Wirtssystemen läßt sich das Protein aufgrund der oben beschriebenen physikochemischen Eigenschaften isolieren.

Die generelle Vorgehensweise zur gentechnischen Herstellung eines neuen Proteins bei bekannter Aminosäurepartialsequenz ist in Lehrbüchern der Gentechnologie, beispielsweise E.L. Winnacker, Gene und Klone, Verlag Chemie, Weinheim, 1984, beschrieben. Die experimentellen Bedingungen für die einzelnen Verfahren wie beispielsweise Anlegen einer Genbank, Hybridisierung, Expression eines Gens sind bei T. Manniatis, "Molecular Cloning", Cold Spring Harbor Laboratory, 1990, beschrieben.

Die in SEQ ID NO 22 beschriebene cDNA-Sequenz bietet die Möglichkeit der Mutagenese des davon codierten Proteins mit Hilfe bekannter Methoden. Dadurch können Proteine hergestellt werden, bei denen einzelne Aminosäuren gegenüber der in SEQ ID NO 22 angegebenen Proteinsequenz ausgetauscht sind. Besonders geeignete Proteine sind solche, die durch Veränderungen der Polypeptidenden, insbesondere durch Verkürzungen der Enden, erhalten werden. Besonders gut geeignete Proteine sind durch Verkürzungen am C-Terminus, bevorzugt um ein bis zwölf Aminosäuren, erhältlich. Die in dieser Weise verkürzten Muteine weisen ebenfalls thrombininhibitorische Wirkung auf.

Die erfindungsgemäßen Proteine werden bevorzugt in Form ihrer pharmazeutisch annehmbaren Salze verwendet.

Die neuen Proteine besitzen blutgerinnungshemmende Eigenschaften. Sie können beispielsweise zur Prophylaxe von Thrombosen oder arteriellen Reokklusionen, zur Behandlung von Thrombosen, zur Konservierung von Blut oder bei der extrakorporalen Zirkulation verwendet werden.

Die neuen Proteine sind wirksame Thrombininhibitoren. Sie können allein oder auch zusammen mit bekannten gerinnungshemmenden Faktoren als Arzneimittel verwendet werden. Als gerinnungshemmende Faktoren werden bevorzugt Thrombininhibitoren, beispielsweise Hirudin, Faktor Xa-Inhibitoren, beispielsweise TAP (Waxman et al., Science 248, 1990, Seite 593-596) oder Plättchen-Aggregationshemmer, beispielsweise Kistrin (Dennis et al., Proc. Natl. Acad. Sci. USA, 87, 1989, Seite 2471-2475) eingesetzt.

Die Erfindung wird durch die folgenden Beispiele weiter veranschaulicht.

### Beispiel 1

### Reinigung der thrombininhibitorischen Proteine aus Landegeln

a) Gewinnung von Egelhomogenaten
150 g lebende Landegel (Haemadipsa sylvestris) wurden in 400 ml 20 mM Natriumphosphatpuffer (pH 7,4) mit einem Mixer bei 4°C 10 Minuten homogenisiert. Das Homogenat wurde 15 Minuten bei 2000 U/min (Sorvall RC-5B, Rotor GS-3) und dann, nach Entfernen des Niederschlags 30 Minuten be- 8000 U/min zentrifugiert. Der Niederschlag wurde verworfen und der Überstand mit 50 mM Tris(hydroxymethyl)-amino-methan/HCL-Puffer pH 8,5 (Tris/HCl-Puffer) auf ein Volumen von etwa 600 ml verdünnt.
Die Proteinlösung hatte ein Volumen von 580 ml, die Proteinkonzentration betrug 4,49 mg/ml und die thrombininhibierende Aktivität betrug 22,2 U/ml.
b) Auftrennung von Landegel-Homogenaten durch Ionenaustausch-Chromatographie
Die aus den Egelhomogenaten erhaltene Proteinlösung wurde auf eine mit 50 mM Tris/HCl-Puffer pH 8,5 äquilibrierte Q-Sepharose®-Säule (50-70 ml, 2,5 cm Durchmesser) appliziert. Nach dem Wegwaschen nicht gebundenen Materials mit dem Äquilibrierungspuffer wurden die gebundenen Proteine mit einem Gradienten von 0 - 1 M NaCl in 20 mM Tris/HCl pH 8,5 eluiert. Es wurden Fraktionen von ca. 7 ml aufgefangen und auf Proteingehalt und thrombininhibitorische Aktivität untersucht (Tabelle 1). Thrombininhibitorische Aktivität enthaltende Fraktionen wurden vereinigt.

**Tabelle 1:**

| Trennung an Q-Sepharose® | | | | | |
|---|---|---|---|---|---|
| | Auftrag | Durchlauf | P₁ | P₂ | P₃ |
| Volumen [ml] | 580 | 580 | 160 | 110 | 130 |
| Proteinkonzentration [mg/ml] | 4,5 | 1,18 | 1,46 | 5,87 | 0,48 |
| Antithrombinaktivität [U/ml] | 22,2 | -- | -- | 78 | 6,5 |

c) Isolierung von Proteinen mit thrombininhibitorischer Aktivität durch Affinitätschromatographie
Wertfraktionen der Q-Sepharose-®-Säule wurden 1:2 mit 20 mM Phosphatpuffer pH 7,5 verdünnt auf eine Thrombin-Sepharose (Herstellung in Beispiel 3 beschrieben) aufgetragen. Nach dem Entfernen nicht gebundener Proteine wurde die Säule zunächst mit 10 Säulenvolumen 20 mM Phosphatpuffer, 500 mM NaCl pH 7,5 gespült. Damit wurden evtl. noch unspezifisch adsorbierte Proteine entfernt. Anschließend wurden die spezifisch gebundenen Thrombin-inhibitoren mit Hilfe von 100 mM Glycin/HCl pH 2,8 eluiert. Die in den Eluaten enthaltene thrombininhibierende Aktivität wurde bestimmt und die Wertfraktionen wurden vereinigt (Tabelle 2). Nach der Konzentrierung mit Hilfe einer 3000 D Membran (Filtron Omega Alpha Cat. No. AM003062) wurde das Konzentrat in einem Vakuum-Verdampfer zur Trockne eingedampft.

**Tabelle 2:**

| Trennung an Thrombin-Sepharose | | | | | |
|---|---|---|---|---|---|
| | Auftrag | Durchlauf | P₁ | P₂ | P₃ |
| Volumen (ml) | 390 | 390 | 21 | 8 | 12 |
| Proteinkonzentration [mg/ml] | 1;47 | 1,18 | 0,03 | -- | 0,035 |
| Antithrombinaktivität [U/ml] | 15,1 | 2 | 36 | 30 | 250 |

d) Reindarstellung der Thrombininhibitorer durch reversed phase HPLC
Die Wertfraktion (P₃) der Thrombinsepharose-Chromatographie wurde in 0,1 gew.-%iger Trifluoressigsäure (TFA) in H₂O gelöst und auf eine reversed phase HPLC Säule (BioRad rp304®) aufgetragen. Nachdem die Säule 5 Minuten mit 0,1 gew.-%iger TFA in Wasser gespült worden war, wurde mit Hilfe eines linearen Gradienten 0,1 gew.-%iger TFA in Wasser/0,1 gew.-%iger TFA in Acetonitril mit 1 %/min aufgetrennt (Fig. 1). Die von der HPLC-Säule eluierenden Proteine werden durch UV-Detektion bei 210 nm erfaßt und fraktioniert. Die in den einzelnen Fraktionen enthaltene thrombininhibierende Aktivität wurde nach Entfernen des Lösungsmittels und Resuspendierung in Wasser (0,2 ml) bestimmt (Tabelle 3). 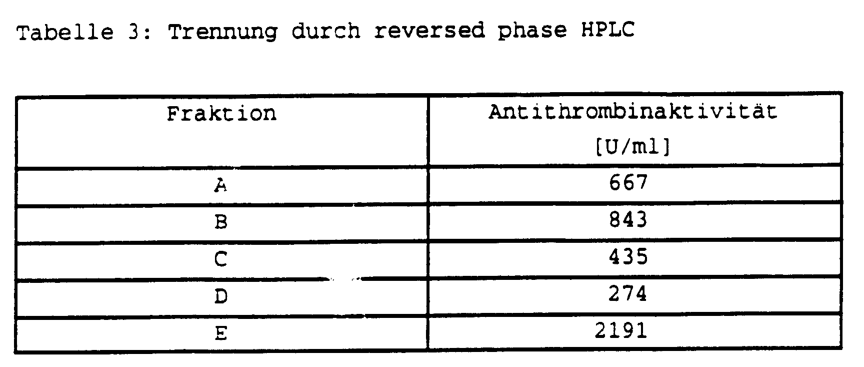 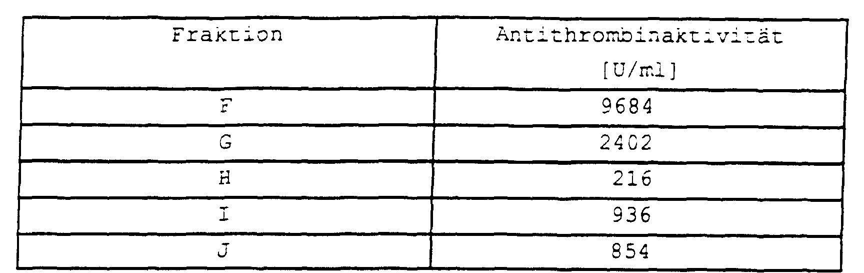
Die aminoterminale Sequenz der thrombininhibierende Aktivität enthaltenden Fraktionen wurde mit Hilfe eines Peptidsequenators (Applied Biosystems, Modell 477A) bestimmt. Tabelle 4 gibt die aminoterminalen Sequenzen der Hauptfraktionen E, F, G und J der Tabelle 3 wieder.

**Tabelle 4:**

| Aminosäuresequenzen der HPLC-Fraktionen | | |
|---|---|---|
| Fraktion | SEQ ID NO | aminoterminale Sequenz |
| E | 4 | IRFGMGKVPCPDGEVGYTCDC (G) EX (I) |
| F | 5 | IRFGMGKVPXPDGEVGYTXDXGEKIXLYGQSXNDGQXS (G) (D) PKX |
| G | 6 | IXFGMGKVPCPDGEVGY (T) (C) (D) (C) (G) XX (I) |
| J | 7 | IXFGMGKVPCLDGEV (G) (Y) |

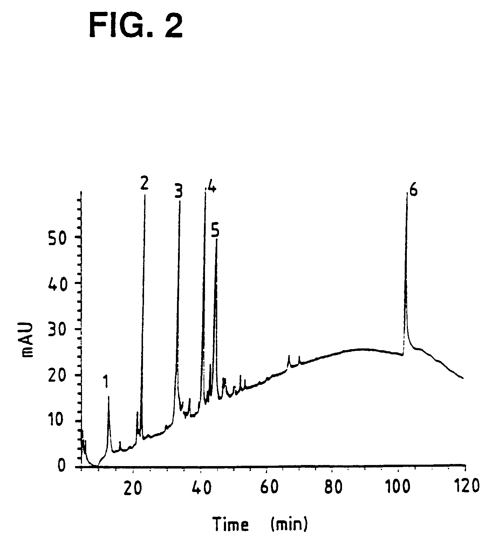

Die in Klammern angegebenen Aminosäuren sind nicht eindeutig identifiziert. X steht für eine Aminosäure, die nicht identifiziert werden konnte.
Peptid-Mapping der isolierten thrombininhibitorischen Proteine
Zur Bestimmung weiterer Aminosäureteilsequenzen wurden die thrombininhibierende Aktivität enthaltenden Fraktionen nach Reduktion und Pyridyl-Ethylierung (Huang et al., Biochemistry, 1989, Vol 28, 661-666) einer Spaltung durch Trypsin-Protease unterworfen. Das Protein/Protease-Verhältnis betrug dabei 20-40 zu 1.
Die Protease Inkubation wurde 4 Stunden bei 37°C nach Angaben des Herstellers durchgeführt. Die erhaltenen Peptidfragmente werden durch reversed phase HPLC an einer C-4-Säule (rp304®, BioRad) aufgetrennt. Dazu wurde, nachdem die Säule 5 Minuten mit 0,5 gew.-%iger TFA in H₂O gespült worden war, ein linearer Gradient von 0,1 gew.-%iger TFA in H₂O in 120 min auf 60 % 0,1 gew.-%ige TFA in Acetonitril verwendet. Die bei 210 nm detektierten Peptide wurden getrennt aufgefangen und nach Abdampfen des Lösungsmittels im Gasphasensequenzer (Applied Biosystems Modell 477A) analysiert. Fig. 2 zeigt die Trennung tryptischer Peptide, erhalten aus dem Verdau der Fraktion F (Fig. 1). Tabelle 5 faßt die nachgewiesenen Aminosäuresequenzen zusammen.

**Tabelle 5:**

| Aminosäuressequenzen der Fraktion F nach Trypsinspaltung | | |
|---|---|---|
| Fraktion | SEQ ID NO | aminoterminale Sequenz |
| 2 | 8 | VPCPDGEVGYTCDCG |
| 3 | 9 | VPCP (D/F) GEVGYTCDCGX (N/D) ICL |
| 4 | 10 | ICIYGQSCNDGQCSGDPKPS (S) X |

X steht für irgendeine natürliche Aminosäure. In Klammern stehen Aminosäuren, die nicht eindeutig identifiziert werden konnten. (D/F) bedeutet, daß an dieser Position sowohl die Aminosäure Asp als auch Phe nachgewiesen wurde.

### Beispiel 2

### Bestimmung der Hemmung von Thrombin durch den Inhibitor

Thrombin (Boehringer Mannheim) wurde zu einer Endkonzentration von (25 mU/ml) in Phosphat gepufferter Salzlösung (PBS) (0,8 g/l NaCl; 0,2 g/l HCl; 0,144 g/l Natriumphosphat; 0,2 g/l Kaliumphosphat, pH 7,5) gelöst.

Chromozym TH (Boehringer Mannheim) wurde in 20 ml H₂O/Flasche gelöst.

50 µl Thrombinlösung und 100 µl Chromozym sowie 25 µl Probe oder Puffer wurden in die Näpfe einer Mikrotiterplatte gegeben. Sofort danach wurde zur Zeit 0 und nach 30 Minuten bei 37°C die Absorption bei 405 nm gemessen.

Bei starker Eigenfarbe der Probe wurde eine weitere Kontrolle ohne Thrombin wie oben behandelt.

Durch die Aktivität des Thrombins wird aus dem chromogenen Farbsubstrat ein bei 405 nm absorbierender Farbstoff freigesetzt. Die Hemmung des Thrombins durch einen Thrombininhibitor ist an einer geringeren Absorptionszunahme bei 405 nm erkennbar und wurde mit Hilfe einer Eichkurve quantifieziert.

### Beispiel 3

### Herstellung einer Affinitätssäule mit Thrombin als Ligand

a) Kopplung:
   6,6 g CNBr aktivierte Sepharose (Pharmacia) wurden mit 200 ml 1 mM HCl auf einer Nutsche gewaschen. Das Gel wurde in 100 mM NaHCO₃, 500 mM NaCl pH 8,3 aufgenommen und sofort mit 10000 Units Thrombin (Sigma) in 100 mM NaHCO₃, 500 mM NaCl, pH 8,3 gemischt.
   Die Lösung wurde 24 Stunden bei 4°C vorsichtig geschüttelt.
b) Blockierung:
   Das Gelmaterial wurde nach Absetzen mit 100 mM NaHCO₃, 500 mM NaCl, pH 8,3 gewaschen. Die Sepharose wurde dann mit 100 mM NaHCO₃, 500 mM NaCl, 1 M Ethanolamin pH 8,3 für 2 Stunden inkubiert.
c) Vorbereitung:
   Zur Entfernung des ungebundenen Thrombin wird das Gelmaterial vor Gebrauch noch einmal in der Säule mit 20 Säulenvolumen PBS pH 7,4 gewaschen.

### Beispiel 4

### Bestimmung des Molekulargewichts durch Molekularsiebchromatographie.

Die Wert fraktionen der Thrombinsepharose-Chromatographie wurden zur Bestimmung des apparenten Molekulargewichtes der gereinigten Thrombininhibitoren auf einer Molekularsiebsäule (Superose 12, Pharmacia) aufgetrennt. Es wurden folgende Chromatographie-Parameter angewandt: Flußrate 0,5 ml/min; Detektion 280 nm; Fraktionsgröße 0,25 ml; Puffer 20 mM phosphatgepufferte Kochsalzlösung (20 mM Natriumphosphat, 0,15 M Natriumchlorid, pH 7,4).

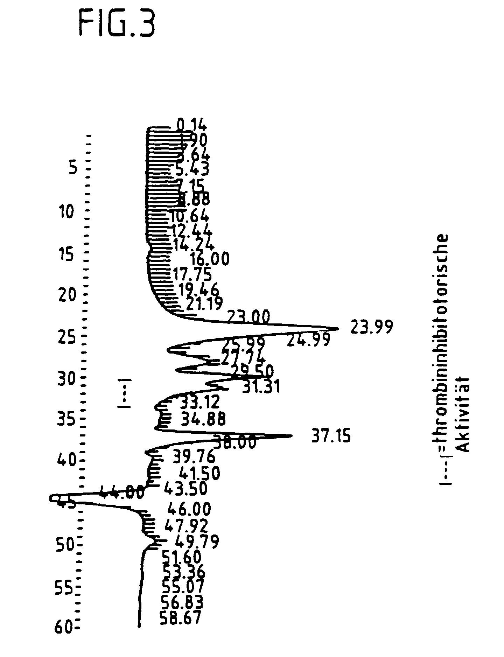

Das erhaltene Chromatogramm ist in Fig. 3 gezeigt. Die thrombininhibitorische Aktivität der Fraktionen wurde wie beschrieben bestimmt und in das Chromatogramm eingetragen. Das Molekulargewicht der Inhibitoren wurde durch Kalibrierung der Säule mit Standard-Proteinen bestimmt und betrug 11200 ± 1000 Dalton.

### Beispiel 5

### Bestimmung des Molekulargewichts durch Tris/Tricine-SDS-Polyacrylamid Gel Elektrophorese.

(Literatur: Analytical Biochemistry, 166, 368 - 379 (1987) Tricine-Sodium Dodecyl Sulfate-Polyacrylamide Gel Electrophoresis for the Separation of Proteins in the range from 1 - 1000 kDa, Schägger, H. and von Jagow, G.)

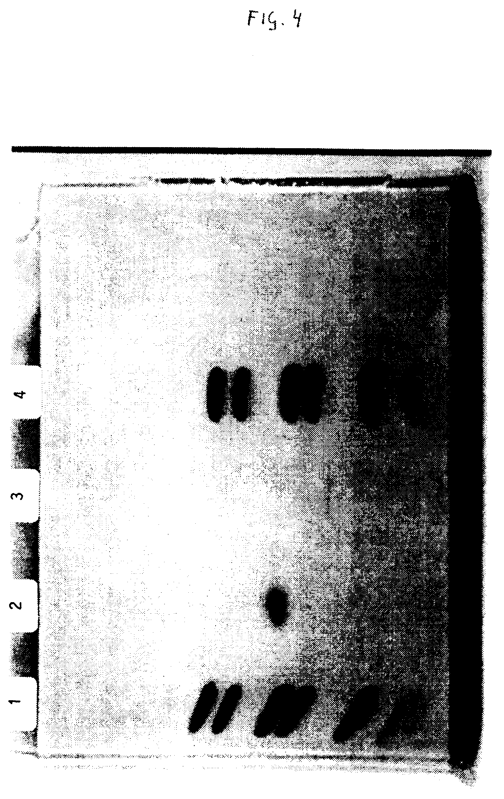

Zur weiteren Charakterisierung der gereinigten Inhibitoren wurde ein Aliquot entsprechend 5 µg auf einem 16 %igen Tris/Tricin-Gel (Bai GmbH, Bensheim) aufgetrennt. Fig. 4 zeigt das Ergebnis nach Färbung des Gels mit Coomassie Brillant Blau. Als Standard fungierten hier Markerproteine (Spur 1 und 4) sowie rekombinantes Hirudin (Spur 3). Das Molekulargewicht der neuen Inhibitoren (Spur 3) wurde mit 5000 ± 1000 Dalton bestimmt.

### Beispiel 6

### Herstellung einer DNA-Sequenz, die für ein thrombininhibitorisches Protein codiert.

### a) Isolierung von RNA und Herstellung einer cDNA-Bank

Gesamt-RNA aus ganzen Tieren der Spezies Haemadipsa sylvestris wurde durch Aufschluß in Guanidiniumthiocyanat gewonnen. Dabei wurde mit Materialien und nach Anleitung des "RNA Isolation Kit" der Firma Stratagene, La Jolla, CA, USA (Catalog Nr. 200345) gearbeitet.

Die polyadenylierte messenger RNA wurde aus der o.a. Gesamt-RNA durch oligo(dT)-Affinitätsseparation selektiert. Dieses Verfahren wurde mit Materialien und nach Anleitung des "PolyATtract mRNA Isolation System" der Firma Promega, Madison, WI, USA (Catalog Nr. Z5200) durchgeführt.

Aus polyadenylierter messenger RNA wurde mit Materialien und nach Anleitung des "ZAP-cDNA Synthesis Kit" der Firma Stratagene, La Jolla, CA, USA (Catalog Nr. 200400) cDNA synthetisiert, die dann mit Materialien und nach Anleitung des "Uni-ZAP XR GigapackII Cloning Kit" der Firma Stratagene, La Jolla, CA, USA (Catalog Nr. 237611) in Lambda Phagen verpackt wurde.

### b) Herstellung von Oligonukleotidproben für die PCR

Zur Klonierung von cDNA-Fragmenten mit Hilfe der Polymerase-Kettenreaktion (PCR, s. "Molecular Cloning", 2nd edition (1989), Sambrook, J. et al., CSH-Press, Seite 14.1 ff.) wurde von Peptiden der in Beispiel 1 beschriebenen ProteinSequenz ausgegangen.

Unter Zugrundelegung des genetischen Codes läßt sich aus der Peptidsequenz: die Nukleinsäuresequenz: und aus der Peptidsequenz: die Nukleinsäuresequenz: des kodierenden DNA-Stranges herleiten. Wegen der bekannten Degeneratior. des genetischen Codes sind an manchen Positionen mehrere Nukleotide (N: A,C,G,T; Y: C,T; R: A,G; H: A,C,T) einsetzbar. Damit ergibt sich für SEQ ID NO: 12 eine 256fache und für SEQ ID NO: 14 eine 384fache Gemischkomplexität. Die genannten Sequenzen wurden als Oligonukleotide synthetisiert.

Folgende Oligonukleotide wurden zusätzlich als 3'Primer synthetisiert: sowie sowie

Die Synthesen wurden mit einem Applied Biosystems Typ 360A DNA-Synthesizer durchgeführt. Die Oligonukleotide wurden nach Entfernung der Schutzgruppen gelelektrophoretisch über ein Acrylamid/Harnstoff-Gel gereinigt.

### c) Herstellung von DNA-templates für die PCR

5 µg Gesamt-RNA oder 1 µg Poly(A)⁺-RNA der unter a) aufgeführten RNA-Präparation wurden mit 1 µg des Oligonukleotides und mit Hilfe des Enzyms Reverse Transkriptase in einzelstrangige cDNA (1°cDNA) übersetzt. Dabei wurde mit Materialien und nach Anleitung des "SuperScript Preamplification System" der Firma Gibco BRL, Eggenstein, Deutschland (Catalog Nr. 80895A) gearbeitet. Nach Beendigung der Reaktion wurden die Syntheseprodukte mittels "Geneclean II Kit" der Firma BIO 101, La Jolla, CA, USA, von kleineren Molekülen und überschüssigen Oligonukleotiden gereinigt.

### d) PCRs und Klonierung einer Teil-cDNA-Sequenz

Die Polymerase-Kettenreaktion wurde nach bekannten Protokollen durchgeführt (s. "Molecular Cloning", 2nd edition (1989), Sambrook, J. et al., CSH-Press, Seite 14.1 ff.). Dazu wurde ein "DNA Thermal Cycler" der Firma Perkin Elmer benutzt. Dabei wurde das Prinzip der "verschachtelten Primer" nach Frohmann, M.A. et al. (Proc. Natl. Acad. Sci. USA (1988) 85, 8998-9002) verwendet und nach Fritz, J.D. et al. (Nucl.Acids Res. (1991) 19, 3747) modifiziert angewendet.

Im einzelnen wurde die 1°cDNA aus c) mit jeweils 20 pmol der Oligonukleotide SEQ ID NO: 12 und SEQ ID NO: 16 amplifiziert. Die Bedingungen waren dabei: 1 min 95°C; 2 min 55°C; 3 min 72°C für 35 Zyklen.

Die PCR-Produkte wurden elektrophoretisch auf einem 1,2%igen Low melting point-Agarose/TBE-Gel aufgetrennt (TBE: 100 mM Tris, 100 mM Borsäure, 2 mM EDTA, pH 8,0).

Aus dem Gel wurden über die gesamte Länge des "Schmiers" etwa 10 Gelscheibchen geschnitten und diese als separate Fraktionen mit DNA-Fragmenten steigender Molmasse geschmolzen.

Aliquots dieser Fraktionen wurden dann separat in eine zweite PCR mit jeweils 20 pmol der Oligonukleotide SEQ ID NO: 14 und SEQ ID NO: 17 eingesetzt. Dabei überschritt der Agaroseanteil nie 1/10 Volumen des PCR-Ansatzes. Reaktionsbedingungen: 1 min 95°C; 2 min 50°C; 3 min 72°C für 35 Zyklen.

Die gelelektrophoretische Auftrennung der Amplifikationsprodukte dieser Fraktionen ließ deutlich eine Reduzierung des komplexen Produktspektrums der ersten PCR hin zu einer definierten Bande nach der zweiten PCR erkennen.

Die solchermaßen selektierten PCR-Produkte wurden nach Standardmethoden eluiert. Nach Subklonierung in die EcoRV-Schnittstelle des Vektors pBluescriptKS und Vermehrung des Plasmides in E.coli DH5alpha ergab die Sequenzanalyse eines Klones (SEQ ID NO: 18) ein offenes Leseraster von 39 Aminosäuren (SEQ ID NO: 19), das mit der vorhergesagten ProteinSequenz übereinstimmte.

### e) PCRs und Klonierung der gesamten codierenden Region

Zur Herleitung der Gesamt-cDNA-Sequenz wurde eine weitere PCR Amplifikation durchgeführt. Als Primer für diese Reaktion wurden die Oligonukleotide: sowie synthetisiert.

Dabei leitet sich SEQ ID NO: 20 aus der in SEQ ID NO: 18 gezeigten Sequenz her; es entspricht der Sequenz des Gegenstranges von Position 158 bis 180. Die zusätzlichen 18 Nukleotide am 5' Ende von SEQ ID NO: 20 sollen eine SalI, bzw. BamHI Schnittstelle rekonstituieren. Oligonukleotid SEQ ID NO: 21 entspricht der Sequenz des käuflichen "reverse" Primers (Stratagene, La Jolla, CA, USA) und ist hergeleitet von Sequenzen des UniZAP XR Lambdaphagen.

Eine PCR mit diesen beiden Oligonukleotiden und einem Aliquot des in a) beschriebenen Gesamt-Phagenlysates als "template" führte zur Isolation einer cDNA-Sequenz (SEQ ID NO: 22), die die komplette codierende Region des o.a. Thrombininhibitors beinhaltet.

Im einzelnen wurden zu diesem Zweck 10 µl eines hochtitrigen Phagenlysates (10⁹-10¹⁰ pfu/ml) der Haemadipsa cDNA-Bank für 10 Minuten gekocht, und in einer PCR Reaktion mit jeweils 20 pmol der Oligonukleotide SEQ ID NO: 20 und SEQ ID NO: 21 amplifiziert. Die Bedingungen waren dabei: i min 95°C; 2 min 55°C; 3 min 72°C für 35 Zyklen; Gesamtvolumen 100 µl.

Das gelelektrophoretisch analysierte PCR-Produkt wurde nach Standardmethoden eluiert. Nach Subklonierung in die EcoRV-Schnittstelle des Vektors pBluescriptKS und Vermehrung des Plasmides in E.coli DH5alpha ergab die Sequenzanalyse eines Klones (SEQ ID NO: 22) ein offenes Leseraster von 77 Aminosäuren (SEQ ID NO: 23), das mit der vorhergesagten ProteinSequenz übereinstimmte.

### f) Heterologe Expression des Thrombininhibitors

Zur Herstellung des rekombinanten Thrombininhibitors wurde zunächst die Aminosäuresequenz SEQ ID NO: 23 von Position 1 bis 57 unter Berücksichtigung der für E. coli typischen Codonauswahl in eine Nukleotidsequenz rückübersetzt. Dabei wurde die folgende Codonauswahl verwendet:

Die doppelsträngige Nukleotidsequenz wurde durch chemische Oligonukleotidsynthese und enzymatische Ligation nach bekannten Verfahren hergestellt. Die Nukleotidsequenz wurde mit XmnI- und BamHI-kompatiblen Enden versehen und in den E. coli Expressionsvektor pMAL-p2 (New England Biolabs) kloniert.

Mit dem rekombinanten Plasmid wurden E. coli DH 5α Zellen transfiziert. Der Thrombininhibitor wurde als Fusionsprotein mit Maltose-Bindungsprotein hergestellt und nach Angaben des Vektor-Herstellers isoliert und gereinigt.

Die Konzentration des periplasmatisch exprimierten Thrombininhibitors betrug 1250 Einheiten pro Liter Kulturmedium.

Legende zu Fig. 1 bis 4
- Fig. 1:: Auftrennung der Wertfraktion (P₃) der Thrombinsepharose durch reversed phase HPLC an einer BioRad rp 304® Säule. Die Probe wurde in 0,1 gew.-%iger Trifluoressigsäure in Wasser gelöst und nach 5 minütigem Spülen mit 0,1 gew.-% Trifluoressigsaure in Wasser mit Hilfe eines linearen Gradienten 0,1 gew.-%ige TFA in Wasser/0,1 gew.-% TFA in Acetonitril mit 1 %/min aufgetrennt. Fraktionen mit thrombininhibitorischer Aktivität (A-J) wurden getrennt gesammelt.
- Fig. 2:: Auftrennung tryptischer Fragmente der Wertfraktion F (Fig. 1) durch reversed phase HPLC. Die aus der Spaltung erhaltenen Peptidfragmente wurden nach Spülen der Säule mit 0,1 gew.-%iger TFA in Wasser für 5 min mit einem linearen Gradienten in 120 min auf 60 % 0,1 %ige TFA/Acetonitril aufgetrennt. Die Detektion erfolgte bei 210 nm und die erhaltenen Fraktionen (1-6) wurden nach Abdampfen des Lösungsmittels im Gasphasensequenzer analysiert.
- Fig. 3:: Bestimmung des apparenten Molekulargewichtes der Thrombininhibitoren durch eine Molekularsiebsäule (Superose 12, Pharmacia). Auftrag: Wertfraktion der Thrombin-Sepharose; Flußrate: 0,5 ml/min; Puffer: 20 mM Natriumphosphat, 0,15 M Natriumchlorid, pH 7,4); Fraktionsgröße 250 µl; Detektion: 280 nm. Die thrombininhibitorische Aktivität enthaltenen Fraktionen sind im Chromatogramm (|---|) eingezeichnet.
- Fig. 4:: Bestimmung de apparenten Molekulargewichts des Thrombininhibitors durch Tris/Tricine-SDS-Gelelektrophorese. Ein Aliquot entsprechend 5 µg wurde auf einem 16 %igen Tris/Tricine-Gel (Bai-GmbH, Bensheim) aufgetrennt und durch Färbung mit Coomassie Brillant Blau sichtbar gemacht (Spur 3). Das Molekulargewicht wurde mit Hilfe von käuflichen Molekulargewichtsmarkern (Spuren 1 und 4) auf 5000±1000 Dalton bestimmt. Ebenfalls aufgetragen wurde rekombinantes Hirudin (Spur 2).

## Patentansprüche

1. Proteine mit thrombininhibitorischer Wirkung aus Landegeln der Gattung Haemadipsa mit der Aminosäuresequenz Ile-Arg-Phe-Gly-Met-Gly-Lys-Val-Pro-Cys-Pro-Asp-Gly-Glu-Val-Gly-Tyr-Thr-Cys-Asp-Cys-Gly-Glu-Lys-Ile-Cys-Leu-Tyr-Gly-Gln-Ser-Cys-Asn-Asp-Gly-Gln-Cys-Ser-Gly-Asp-Pro-Lys-Pro-Ser-Ser-Glu-Phe-Glu-Glu-Phe-Glu-Ile-Asp-Glu-Glu-Glu-Lys (SEQ ID NO: 1) oder einer Aminosäuresequenz, die durch C-terminale Verkürzung von SEQ ID NO: 1 um ein bis zwölf Aminosäuren erhalten wird.

2. Proteine nach Anspruch 1 mit der Sequenz SEQ ID NO: 2.

3. DNA-Sequenzen, die für Proteine mit thrombininhibitorischer Wirkung codieren, und die aus der Gruppe, die von
a) DNA-Sequenzen der in SEQ ID NO 22 beschriebenen Struktur,
b) DNA-Sequenzen, die für Proteine gemäß Anspruch 1 oder 2 codieren, und
c) DNA-Sequenzen, die unter Standardbedingungen mit DNA-Sequenzen a) oder b) hydridisieren, gebildet wird,
ausgewählt sind.

4. Expressionsvektor, der eine DNA-Sequenz gemäß Anspruch 3 enthält.

5. Verwendung von Proteinen, die von DNA-Sequenzen gemäß Anspruch 3 codiert werden, zur Herstellung von Arzneimitteln für die Bekämpfung von Krankheiten.

6. Arzneimittel, enthaltend ein oder mehrere Proteine, die von DNA-Sequenzen gemäß Anspruch 3 codiert werden und einen weiteren gerinnungsinhibierenden Faktor.

7. Arzneimittel nach Anspruch 6, dadurch gekennzeichnet, daß als weiterer gerinnungsinhibierender Faktor ein Hirudin verwendet wird.

## Claims

1. A protein with thrombin-inhibitory action from terrestrial leeches of the genus Haemadipsa with the amino-acid sequence Ile-Arg-Phe-Gly-Met-Gly-Lys-vat-Pro-Cys-Pro-Asp-Gly-Glu-Val-Gly-Tyr-Thr-Cys-Asp-Cys-Gly-Glu-Lys-Ile-Cys-Leu-Tyr-Gly-Gln-Ser-Cys-Asn-Asp-Gly-Gln-Cys-Ser-Gly-Asp-Pro-Lys-Pro-Ser-Ser-Glu-Phe-Glu-Glu-Phe-Glu-Ile-Asp-Glu-Glu-Glu-Lys (SEQ ID NO: 1) or an amino-acid sequence which is obtained by C-terminal truncation of SEQ ID NO: 1 by from one to twelve amino acids.

2. A protein as claimed in claim 1 with the sequence SEQ ID NO: 2.

3. A DNA sequence which codes for a protein with thrombin-inhibitory action and which is selected from the group comprising
a) DNA sequences of the structure described in SEQ ID NO: 22,
b) DNA sequences which code for proteins as claimed in claim 1 or 2, and
c) DNA sequences which hybridize under standard conditions with DNA sequences a) or b).

4. An expression vector which contains a DNA sequence as claimed in claim 3.

5. The use of proteins which are encoded by DNA sequences as claimed in claim 3 for producing drugs for controlling diseases.

6. A drug containing one or more proteins which are encoded by DNA sequences as claimed in claim 3, and another coagulation-inhibiting factor.

7. A drug as claimed in claim 6, wherein a hirudin is used as another coagulation-inhibiting factor.

## Revendications

1. Protéines à activité inhibitrice de la thrombine provenant de sangsues terrestres de l'espèce Haemadipsa avec la séquence d'aminoacides Ile-Arg-Phe-Gly-Met-Gly-Lys-Val-Pro-Cys-Asp-Gly-Glu-Val-Gly-Tyr-Thr-Cys-Asp-Cys-Gly-Glu-Lys-Ile-Cys-Leu-Tyr-Gly-Gln-Ser-Cys-Asn-Asp-Gly-Gln-Cys-Ser-Gly-Asp-Pro-Lys-Pro-Ser-Ser-Glu-Phe-Glu-Glu-Phe-Glu-Ile-Asp-Glu-Glu-Glu-Lys, (SEQ ID N°: 1), ou une séquence d'aminoacides qui a été obtenue par raccourcissement C-terminal, de la SEQ ID N°: 1 d'un à douze aminoacides.

2. Protéines suivant la revendication 1 avec la séquence SEQ ID N°: 2.

3. Séquences d'ADN qui codent pour des protéines à activité inhibitrice de la thrombine et qui sont choisies dans le groupe formé par
a) les séquences d'ADN de la structure décrite dans SEQ ID N°: 22,
b) les séquences d'ADN qui codent pour les protéines suivant la revendication 1 ou 2 et
c) les séquences d'ADN qui, dans des conditions normales, sont formées avec hybridation aux séquences d'ADN a) ou b).

4. Vecteur d'expression qui contient une séquence d'ADN suivant la revendication 3.

5. Utilisation de protéines qui sont codées par des séquences d'ADN suivant la revendication 3, en vue de la préparation de médicaments destinés à lutter contre des maladies.

6. Médicament qui contient une ou plusieurs protéines qui sont codées par des séquences d'ADN suivant la revendication 3 et un autre facteur inhibant la coagulation sanguine.

7. Médicament suivant la revendication 6, caractérisé en ce que l'on utilise de l'hirudine, à titre d'autre facteur inhibant la coagulation sanguine.


### SEQUENCE LISTING

### (1) GENERAL INFORMATION:

(i) APPLICANT:
   (A) NAME: BASF Aktiengesellschaft
   (B) STREET: Carl-Bosch-Strasse 38
   (C) CITY: Ludwigshafen
   (E) COUNTRY: Bundesrepublik Deutschland
   (F) POSTAL CODE (ZIP): D-6700
   (G) TELEPHONE: 0621/6048526
   (H) TELEFAX: 0621/6043123
   (I) TELEX: 1762175170
(ii) TITLE OF INVENTION:
   Neue thrombininhibitorische Proteine aus Landblutegeln
(iii) NUMBER OF SEQUENCES: 23
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(v) CURRENT APPLICATION DATA:
   APPLICATION NUMBER:

### (2) INFORMATION FOR SEQ ID NO:1:

(i) SEQUENCE CHARACTERISTICS:
   (A) LENGTH: 57 amino acids
   (B) TYPE: amino acid
   (D) TOPOLOGY: linear
(ii) MOLECULE TYPE: protein
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animals
(ix) FEATURE:
   (A) NAME/KEY: Protein
   (B) LOCATION: 1..57
(xi) SEQUENCE DESCRIPTION: SEQ ID NO:1:
   Ile Arg Phe Gly Met Gly Lys Val Pro Cys Pro Asp Gly Glu Val Gly
   1               5                   10                  15
   Tyr Thr Cys Asp Cys Gly Glu Lys Ile Cys Leu Tyr Gly Gln Ser Cys
               20                  25                  30
   Asn Asp Gly Gln Cys Ser Gly Asp Pro Lys Pro Ser Ser Glu Phe Glu
           35                  40                  45
   Glu Phe Glu Ile Asp Glu Glu Glu Lys
       50                  55

### (2) INFORMATION FOR SEQ ID NO:2:

(i) SEQUENCE CHARACTERISTICS:
   (A) LENGTH: 45 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animal
(ix) FEATURE:
   (A) NAME/KEY: Peptide
   (B) LOCATION: 1..45
(xi) SEQUENCE DESCRIPTION: SEQ ID NO:2:
   Ile Arg Phe Gly Met Gly Lys Val Pro Cys Pro Asp Gly Glu Val Gly
   1               5                   10                  15
   Tyr Thr Cys Asp Cys Gly Glu Lys Ile Cys Leu Tyr Gly Gln Ser Cys
               20                  25                  30
   Asn Asp Gly Gln Cys Ser Gly Asp Pro Lys Pro Ser Ser
           35                  40                  45

### (2) INFORMATION FOR SEQ ID NO:3:

(i) SEQUENCE CHARACTERISTICS:
   (A) LENGTH: 44 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animal
(ix) FEATURE:
   (A) NAME/KEY: Peptide
   (B) LOCATION: 1..44
(ix) FEATURE:
   (A) NAME/KEY: Modified-site
   (B) LOCATION: 11
   (D) OTHER INFORMATION: /label= ambiguity
      /note= "Xaa is Pro or Leu"
(ix) FEATURE:
   (A) NAME/KEY: Modified-site
   (B) LOCATION: 12
   (D) OTHER INFORMATION: /label= ambiguity
      /note= "Xaa is Asp or Phe"
(ix) FEATURE:
   (A) NAME/KEY: Modified-site
   (B) LOCATION: 23
   (D) OTHER INFORMATION: /label= ambiguity
      /note= "Xaa is Glu or Trp"
(ix) FEATURE:
   (A) NAME/KEY: Modified-site
   (B) LOCATION: 24
   (D) OTHER INFORMATION: /label= ambiguity
      /note= "Xaa is Lys, Asn or Asp"
(xi) SEQUENCE DESCRIPTION: SEQ ID NO:3:
   Ile Arg Phe Gly Met Gly Lys Val Pro Cys Xaa Xaa Gly Glu Val Gly
   1               5                   10                  15
   Tyr Thr Cys Asp Cys Gly Xaa Xaa Ile Cys Leu Tyr Gly Gln Ser Cys
               20                  25                  30
   Asn Asp Gly Gln Cys Ser Gly Asp Pro Lys Pro Ser
           35                  40

### (2) INFORMATION FOR SEQ ID NO:4:

(i) SEQUENCE CHARACTERISTICS:
   (A) LENGTH: 25 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..25
(ix) FEATURE:
   (A) NAME/KEY: Modified-site
   (B) LOCATION: 22
   (D) OTHER INFORMATION: /label= unverified
      /note= "position unverified"
(ix) FEATURE:
   (A) NAME/KEY: Modified-site
   (B) LOCATION: 24
   (D) OTHER INFORMATION: /label= unidentified
      /note= "position is unidentified"
(ix) FEATURE:
   (A) NAME/KEY: Modified-site
   (B) LOCATION: 25
   (D) OTHER INFORMATION: /label= unverified
      /note= "position is not verified"
(xi) SEQUENCE DESCRIPTION: SEQ ID NO:4:
   Ile Arg Phe Gly Met Gly Lys Val Pro Cys Pro Asp Gly Glu Val Gly
   1               5                   10                  15
   Tyr Thr Cys Asp Cys Gly Glu Xaa Ile
               20                  25

### (2) INFORMATION FOR SEQ ID NO:5:

(i) SEQUENCE CHARACTERISTICS:
   (A) LENGTH: 43 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..43
(ix) FEATURE:
   (A) NAME/KEY: Modified-site
   (B) LOCATION: 1..43
   (D) OTHER INFORMATION: /label= unidentified
      /note= "Xaa: positions are not identified"
(ix) FEATURE:
   (A) NAME/KEY: Modified-site
   (B) LOCATION: 39..40
   (D) OTHER INFORMATION: /label= unverified
      /note= "positions are not verified"
(xi) SEQUENCE DESCRIPTION: SEQ ID NO:5:
   Ile Arg Phe Gly Met Gly Lys Val Pro Xaa Pro Asp Gly Glu Val Gly
   1               5                   10                  15
   Tyr Thr Xaa Asp Xaa Gly Glu Lys Ile Xaa Leu Tyr Gly Gln Ser Xaa
               20                  25                  30
   Asn Asp Gly Gln Xaa Ser Gly Asp Pro Lys Xaa
           35                  40

### (2) INFORMATION FOR SEQ ID NO:6:

(i) SEQUENCE CHARACTERISTICS:
   (A) LENGTH: 25 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..25
(ix) FEATURE:
   (A) NAME/KEY: Modified-site
   (B) LOCATION: 1..25
   (D) OTHER INFORMATION: /label= unidentified
      /note= "Xaa: positions are not identified"
(ix) FEATURE:
   (A) NAME/KEY: Modified-site
   (B) LOCATION: 18..22
   (D) OTHER INFORMATION: /label= unverified
      /note= "positions are not verified"
(ix) FEATURE:
   (A) NAME/KEY: Modified-site
   (B) LOCATION: 25
   (D) OTHER INFORMATION: /label= unverified
      /note= "position is not verified"
(xi) SEQUENCE DESCRIPTION: SEQ ID NO:6:
   Ile Xaa Phe Gly Met Gly Lys Val Pro Cys Pro Asp Gly Glu Val Gly
   1               5                   10                  15
   Tyr Thr Cys Asp Cys Gly Xaa Xaa Ile
               20                  25

### (2) INFORMATION FOR SEQ ID NO:7:

(i) SEQUENCE CHARACTERISTICS:
   (A) LENGTH: 17 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..17
(ix) FEATURE:
   (A) NAME/KEY: Modified-site
   (B) LOCATION: 2
   (D) OTHER INFORMATION: /label= unidentified
      /note= "position is not identified"
(ix) FEATURE:
   (A) NAME/KEY: Modified-site
   (B) LOCATION: 16..17
   (D) OTHER INFORMATION: /label= unverified
      /note= "positions are not verified"
(xi) SEQUENCE DESCRIPTION: SEQ ID NO:7:
   Ile Xaa Phe Gly Met Gly Lys Val Pro Cys Leu Asp Gly Glu Val Gly
   1               5                   10                  15
   Tyr

### (2) INFORMATION FOR SEQ ID NO:8:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..15
(xi) SEQUENCE DESCRIPTION: SEQ ID NO:8:
   Val Pro Cys Pro Asp Gly Glu Val Gly Tyr Thr Cys Asp Cys Gly
   1               5                   10                  15

### (2) INFORMATION FOR SEQ ID NO:9:

(i) SEQUENCE CHARACTERISTICS:
   (A) LENGTH: 20 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..20
(ix) FEATURE:
   (A) NAME/KEY: Modified-site
   (B) LOCATION: 5
   (D) OTHER INFORMATION: /label= ambiguity
      /note= "position 5 was also identified as Phe"
(ix) FEATURE:
   (A) NAME/KEY: Modified-site
   (B) LOCATION: 17
   (D) OTHER INFORMATION: /label= ambiguity
      /note= "position 17 was also identified as Asp"
(ix) FEATURE:
   (A) NAME/KEY: Modified-site
   (B) LOCATION: 16
   (D) OTHER INFORMATION: /label= unidentified
      /note= "position is not identified"
(xi) SEQUENCE DESCRIPTION: SEQ ID NO:9:
   Val Pro Cys Pro Asp Gly Glu Val Gly Tyr Thr Cys Asp Cys Gly Xaa
   1               5                   10                  15
   Asn Ile Cys Leu
               20

### (2) INFORMATION FOR SEQ ID NO:10:

(i) SEQUENCE CHARACTERISTICS:
   (A) LENGTH: 22 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
   (A) ORGANISM: Haemadipsa sylvestris
   (B) STRAIN: Blanchard
(ix) FEATURE:
   (A) NAME/KEY: Peptide
   (B) LOCATION: 1..22
(ix) FEATURE:
   (A) NAME/KEY: Modified-site
   (B) LOCATION: 21
   (D) OTHER INFORMATION: /label= unverified
      /note= "position is not verified"
(ix) FEATURE:
   (A) NAME/KEY: Modified-site
   (B) LOCATION: 22
   (D) OTHER INFORMATION: /label= unidentified
      /note= "position is not identified"
(xi) SEQUENCE DESCRIPTION: SEQ ID NO:10:
   Ile Cys Leu Tyr Gly Gln Ser Cys Asn Asp Gly Gln Cys Ser Gly Asp
   1               5                   10                  15
   Pro Lys Pro Ser Ser Xaa
               20

### (2) INFORMATION FOR SEQ ID NO:11:

(i) SEQUENCE CHARACTERISTICS:
   (A) LENGTH: 8 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
   (A) ORGANISM: Haemadipsa sylvestris
   (B) STRAIN: Blanchard
   (D) DEVELOPMENTAL STAGE: Adult
   (F) TISSUE TYPE: whole animals
(xi) SEQUENCE DESCRIPTION: SEQ ID NO:11:
   Gly Met Gly Lys Val Pro Cys Pro
   1               5

### (2) INFORMATION FOR SEQ ID NO:12:

(i) SEQUENCE CHARACTERISTICS:
   (A) LENGTH: 23 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
   (A) ORGANISM: Haemadipsa sylvestris
   (B) STRAIN: Blanchard
(xi) SEQUENCE DESCRIPTION: SEQ ID NO:12:
   GGAATGGGNA ARGTNCCNTG YCC                                   23

### (2) INFORMATION FOR SEQ ID NO:13:

(i) SEQUENCE CHARACTERISTICS:
   (A) LENGTH: 8 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animals
(xi) SEQUENCE DESCRIPTION: SEQ ID NO:13:
   Cys Asp Cys Gly Glu Lys Ile Cys
   1               5

### (2) INFORMATION FOR SEQ ID NO:14:

(i) SEQUENCE CHARACTERISTICS:
   (A) LENGTH: 23 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
   (A) ORGANISM: Haemadipsa sylvestris
   (B) STRAIN: Blanchard
(xi) SEQUENCE DESCRIPTION: SEQ ID NO:14:
   TGYGAYTGYG GNGARAARAT HTG 23

### (2) INFORMATION FOR SEQ ID NO:15:

(i) SEQUENCE CHARACTERISTICS:
   (A) LENGTH: 45 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:15:
   CGAGGGGGAT GGTCGACGGA AGCGACCTTT TTTTTTTTTT TTTTT 45

### (2) INFORMATION FOR SEQ ID NO:16:

(i) SEQUENCE CHARACTERISTICS:
   (A) LENGTH: 19 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:16:
   CGAGGGGGAT GGTCGACGG 19

### (2) INFORMATION FOR SEQ ID NO:17:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:17:
   GATGGTCGAC GGAAGCGACC 20

### (2) INFORMATION FOR SEQ ID NO:18:

(i) SEQUENCE CHARACTERISTICS:
   (A) LENGTH: 277 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animals
(vii) IMMEDIATE SOURCE:
   (B) CLONE: pBSKS/139.101#6
(ix) FEATURE:
   (A) NAME/KEY: CDS
   (B) LOCATION: 1..120
(ix) FEATURE:
   (A) NAME/KEY: 3'UTR
   (B) LOCATION: 121..257
(xi) SEQUENCE DESCRIPTION: SEQ ID NO:18:
   TGT GAT TGT GGT GAA AAG ATA TGT TTG TAT GGA CAA AGC TGC AAT GAT        48
   Cys Asp Cys Gly Glu Lys Ile Cys Leu Tyr Gly Gln Ser Cys Asn Asp
     1               5                  10                  15
   GGT CAA TGC TCA GGT GAT CCT AAA CCA AGC AGT GAA TTC GAA GAA TTT        96
   Gly Gln Cys Ser Gly Asp Pro Lys Pro Ser Ser Glu Phe Glu Glu Phe
                20                  25                  30
   GAA ATT GAT GAA GAA GAA AAA TAATTATTAA TATTTCCTAG TATCTCTAGT         147
   Glu Ile Asp Glu Glu Glu Lys
            35                  40
   ACAATTGTTA GCTTTGGCAA TAATTATCTG TAACAAATGA ATTGTTTGAA AAATAATAAA    207
   GAATTATTAT TATTGATGAC AAAAAAAAAA AAAAAAAAAA AAAAAAAAAA GGTCGCTTCC    267
   GTCGACCATC                                                           277

### (2) INFORMATION FOR SEQ ID NO:19:

(i) SEQUENCE CHARACTERISTICS:
   (A) LENGTH: 39 amino acids
   (B) TYPE: amino acid
   (D) TOPOLOGY: linear
(ii) MOLECULE TYPE: protein
(xi) SEQUENCE DESCRIPTION: SEQ ID NO:19:
   Cys Asp Cys Gly Glu Lys Ile Cys Leu Tyr Gly Gln Ser Cys Asn Asp
     1               5                  10                  15
   Gly Gln Cys Ser Gly Asp Pro Lys Pro Ser Ser Glu Phe Glu Glu Phe
                20                  25                  30
   Glu Ile Asp Glu Glu Glu Lys
            35

### (2) INFORMATION FOR SEQ ID NO:20:

(i) SEQUENCE CHARACTERISTICS:
   (A) LENGTH: 42 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(iv) ANTI-SENSE: NO
(vi) ORIGINAL SOURCE:
   (A) ORGANISM: Haemadipsa sylvestris
   (B) STRAIN: Blanchard
(xi) SEQUENCE DESCRIPTION: SEQ ID NO:20:
   GGGGGGGTCG ACGGATCCGT TACAGATAAT TATTGCCAAA GC                42

### (2) INFORMATION FOR SEQ ID NO:21:

(i) SEQUENCE CHARACTERISTICS:
   (A) LENGTH: 18 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(iv) ANTI-SENSE: NO
(xi) SEQUENCE DESCRIPTION: SEQ ID NO:21:
   CAGGAAACAG CTATGACC                                           18

### (2) INFORMATION FOR SEQ ID NO:22:

(i) SEQUENCE CHARACTERISTICS:
   (A) LENGTH: 353 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Haemadipsa sylvestris
   (B) STRAIN: Blanchard
   (D) DEVELOPMENTAL STAGE: adult
   (F) TISSUE TYPE: whole animals
(vii) IMMEDIATE SOURCE:
   (B) CLONE: pBSKS/rev.148#5
(ix) FEATURE:
   (A) NAME/KEY: 5'UTR
   (B) LOCATION: 1..40
(ix) FEATURE:
   (A) NAME/KEY: CDS
   (B) LOCATION: 41..274
(ix) FEATURE:
   (A) NAME/KEY: sig_peptide
   (B) LOCATION: 41..100
(ix) FEATURE:
   (A) NAME/KEY: mat_peptide
   (B) LOCATION: 101..274
(ix) FEATURE:
   (A) NAME/KEY: 3'UTR
   (B) LOCATION: 275..335
(xi) SEQUENCE DESCRIPTION: SEQ ID NO:22:
   ATTTAGTTAA TTTTATTCTG CTTTGAAAGA ATTGCTGAAG ATG TTC TCA ACA AAG       55
                                               Met Phe Ser Thr Lys
                                               -20
   ATG TTT GTT GTT TTT GTT GCA GTT TGC ATC TGC GTA ACT CAG TCT ATA      103
   Met Phe Val Val Phe Val Ala Val Cys Ile Cys Val Thr Gln Ser Ile
   -15                 -10                  -5                   1
   AGA TTT GGA ATG GGC AAA GTT CCA TGC CCA GAT GGC GAA GTG GGA TAC      151
   Arg Phe Gly Met Gly Lys Val Pro Cys Pro Asp Gly Glu Val Gly Tyr
                 5                  10                  15
   ACT TGC GAC TGT GGG GAA AAG ATT TGT TTG TAT GGA CAA AGC TGC AAT      199
   Thr Cys Asp Cys Gly Glu Lys Ile Cys Leu Tyr Gly Gln Ser Cys Asn
            20                  25                  30
   GAT GGT CAA TGC TCA GGT GAT CCT AAA CCA AGC AGT GAA TTC GAA GAA      247
   Asp Gly Gln Cys Ser Gly Asp Pro Lys Pro Ser Ser Glu Phe Glu Glu
        35                  40                  45
   TTT GAA ATT GAT GAA GAA GAA AAA TAATTATTAA TATTTCCTAG TATCTCTAGT     301
   Phe Glu Ile Asp Glu Glu Glu Lys
    50                  55
   ACAATTGTTA GCTTTGGCAA TAATTATCTG TAACGGATCC GTCGACCCCC CC            353

### (2) INFORMATION FOR SEQ ID NO:23:

(i) SEQUENCE CHARACTERISTICS:
   (A) LENGTH: 77 amino acids
   (B) TYPE: amino acid
   (D) TOPOLOGY: linear
(ii) MOLECULE TYPE: protein
(xi) SEQUENCE DESCRIPTION: SEQ ID NO:23:
   Met Phe Ser Thr Lys Met Phe Val Val Phe Val Ala Val Cys Ile Cys
   -20                 -15                 -10                  -5
   Val Thr Gln Ser Ile Arg Phe Gly Met Gly Lys Val Pro Cys Pro Asp
                        1               5                  10
   Gly Glu Val Gly Tyr Thr Cys Asp Cys Gly Glu Lys Ile Cys Leu Tyr
            15                  20                  25
   Gly Gln Ser Cys Asn Asp Gly Gln Cys Ser Gly Asp Pro Lys Pro Ser
        30                  35                  40
   Ser Glu Phe Glu Glu Phe Glu Ile Asp Glu Glu Glu Lys
    45                  50                  55